# EUROPEAN PATENT APPLICATION

(11) **EP 3 725 899 A1**
(43) Date of publication of application: **21.10.2020**
(21) Application number: 19382299.6
(22) Date of filing: 16.04.2019
(51) Int. Cl.: C12Q 1/6886

(54) **METHOD FOR DETERMINING THE PERCENTAGE OF METHYLATION OF THE PROMOTER OF THE GENE O6-METHYLGUANINE-DNA METHYLTRANSFERASE (MGMT) IN CIRCULATING EXOSOMES**

(71) Applicant: Fundación para la Investigación Biomédica del Hospital Universitario de la Paz (FIBHULP), 28046 Madrid (ES)
(72) Inventor: IBÁÑEZ DE CÁCERES, Inmaculada, 28046 Madrid (ES); DE CASTRO CARPEÑO, Javier, 28046 Madrid (ES); ROSAS ALONSO, Rocío, 28046 Madrid (ES); PERNÍA ARIAS, Olga, 28046 Madrid (ES); MARTÍNEZ, Virginia, 28046 Madrid (ES); ESTEBAN RODRÍGUEZ, Mª Isabel, 28046 Madrid (ES)
(74) Representative: Hoffmann Eitle

(57) **Abstract**

In the present invention a methodology has been identified for the detection of MGMT methylation in blood, serum or plasma of a human subject with high sensitivity and specificity, surpassing the techniques described so far. This methodology will allow the diagnosis and monitoring of those patients diagnosed with glioblastomas using a non-invasive method. This invention could be extended to other tumor types in which methylation of the MGMT gene was also present as a tumor marker.

## Description

### Technical field of the invention

This invention refers to the medical field, in particular, the prediction of progression or response to treatment of a patient affected by a glioblastoma; more particularly the *in vitro* use of DNA molecules isolated from the inside of circulating exosomes of a sample isolated from serum, blood or plasma obtained or isolated from a human subject, to determine the percentage of methylation in that DNA of the promoter of the O6-methylguanine-DNA methyltransferase (MGMT) gene.

### Background of the invention

Glioblastoma (GB) is the most common primary malignant central nervous system (CNS) tumor in adults (accounting for more than 50%) and is invariably associated with poor prognosis. Only 33% of patients survive a year and 5% of patients live more than 5 years after diagnosis. The World Health Organization (WHO) classifies gliomas primarily by histopathological criteria in: astrocytomas, oligodendrogliomas, oligoastrocytomas and ependymomas. In addition, it establishes a gradation related to disease prognosis that identifies those with high-grade as grade III (anaplastic astrocytoma) and grade IV (GB) astrocytomas.

GBs can compromise any neuroanatomical structure, but in adults it is more common in the cerebral hemispheres, while in children it is in the posterior fossa. Its infiltrating growth is extremely rapid. Histologically, it is composed of cells of great morphological variability: bizarre, pleomorphic and multinucleated; with high mitotic activity; microvascular proliferation; severe and characteristic endothelial hyperplasia; intravascular microthrombosis, and extensive ischemic necrosis or in the form of pseudopalisading. The name "Glioblastoma Multiforme" is due to the great heterogeneity that characterizes the tumor with varied cytological features and patterns. In general, they show well-differentiated foci alternating with poorly differentiated foci. Traditionally, they have been classified into two morphologically identical subtypes: primary (GB1) and secondary (GB2).

Glioblastomas GB1 represent the majority of GB, appear without precedent lesions and are more frequent in advanced age (average age of 60 years). The evolution is quick due to the rapid growth of the tumour. Glioblastomas GB2 represent less than 10% of the GB, arise as progression of low-grade astrocytomas (diffuse astrocytoma or anaplastic astrocytoma) and affect young patients. Their evolution is slower, and average survival is significantly higher due to the younger age at which the disease appears and slower tumor growth. Both entities are distinct diseases that evolve through different molecular pathways.

The WHO includes Giant Cell Glioblastoma and Gliosarcoma within the denomination of GB, as varieties with some characteristic traits. In addition, entities with their own characteristics have been described and are currently being reviewed by the WHO. The Small Cell Glioblastoma is characterized by a cytological predominance of small elements with a high nucleus/cytoplasm quotient and a high cellular proliferation index, corresponding to 10% of the total GB, which is associated with a worse prognosis. The GB with primitive neuroectodermal tumour-like components (GBM/PNET) is an infrequent entity -more common in children and young adults- probably associated with a favorable prognosis, and is characterized by protein expression CD56-positive and vimentine-negative. The GB with an oligodendroglial component is also an uncommon entity with a yet unknown prognosis.

Additionally, the integrated genomic profiling of GB developed with studies from TheCancerGenome Atlas (TCGA) research network has revealed several molecular subtypes, these subtypes are defined by their most important gene expression alterations, and are associated with specific mutations and copy-number alterations. The following GB subtypes are recognized: classic type, mesenchymal type, proneural type and neural type (Louis DN, Ohgaki H, Wiestler OD, Cavenee WK, Burger PC, Jouvet A, et al. The 2007 WHO classification of tumours of the central nervous system.ActaNeuropathol.2007 Aug;114(2):97- 109; Liu W, Zhang S, Zhang L, Cui Q, Wang J, Gui T, et al. A prognostic analysis of pediatrics central nervous system small cell tumors: evaluation of EGFR family gene amplification and overexpression. DiagnPathol. 2014 Jul 1;9:132).

The poor prognosis associated with this malignancy makes it a public health problem. Knowledge of the biological and molecular basis of the disease is crucial for a better understanding of this entity, daily management of patients and for the development of more effective treatment strategies. In any case, studies on predictive markers of response to alkylating agents have identified the enzyme O6-methylguanine-DNA methyltransferase (MGMT) as responsible for the elimination of alkyl groups from the O6 position of guanine (effect of alkylating agents). The MGMT promoter lacks regulatory elements known as TATA box or CAT box, and contains a CpG island rich in 97 CG dinucleotides or CpG sites, located in the MGMT 5' region. Methylated CpG sites bind to specific proteins and this complex causes altered chromatin structures and loss of transcription (MGMT silencing). The MGMT promoter is methylated in about 45% of glioblastomas and is more common in secondary glioblastomas. The study of choice for evaluating MGMT promoter methylation is by Methylation-Specific PCR (MSP) or by pyrosequencing. Evaluating MGMT protein expression with immunohistochemical techniques is not recommended.

In the evaluation of MGMT promoter methylation, the DNA of glioblastoma tumors is subjected to bisuphite reaction, whereby non-methylated cytosines residues are converted to uracils after treatment. A detailed explanation of this technique is described in the *Materials and Methods* section of this specification. Methylation of the MGMT promoter is associated with increased survival regardless of the treatment chosen. It is also associated with a greater response to temozolomide, radiotherapy (Olson RA, Brastianos PK, Palma DA. Prognostic and predictive value of epigenetic silencing of MGMT in patients with high grade gliomas: a systematic review and meta-analysis. J Neurooncol. 2011 Nov;105(2):325-35) and the combination of both. Thus, a study evaluating the methylation status of the MGMT promoter in 206 tumors was conducted, finding that the presence of methylation was associated with increased overall survival. In addition, only those patients with methylation showed an increase in progression-free survival with the use of concurrent radiotherapy/tempozolomide treatment (p<0.001). Finally, the Phase III clinical trial NOA-08 evaluated the use of temozolomide monotherapy or radiation in 373 patients over 65 years of age and no difference in survival was found. The tumor MGMT promoter methylation test performed on 209 patients identified those with the greatest increase in progression-free survival in the group receiving temozolomide, but failed to predict prognosis among those receiving radiation.

Additionally, it is known that less than 10% of patients with gliomas harbor detectable free circulating tumor DNA (ctDNA). This is because gliomas are tumors that occur in the brain and because of their anatomical location they are protected by the blood-brain barrier (BBB). BBB is a protection system formed by endothelial cells that cover the capillaries of the brain. It serves to control and restrict the passage of molecules between the blood circulation and the cerebrospinal fluid. This physical barrier, in addition to restricting the passage of toxic substances into the brain, limits the free circulation of ctDNA, which explains its low blood levels. Therefore, determination of the methylation status of the MGMT promoter to evaluate response to temozolomide, radiation therapy, or both, is currently restricted to the use of tumor samples.

On the other hand, some studies evaluated other prognostic or response to treatment markers, including the presence of an oligodendroglial component. Oligodendroglial tumors have been previously associated with the loss of genetic material from the 1p and 19q chromosomal arms that apparently cause the loss of the tumor suppressor genes CIC (chromosome 19q) and FUBP-1 (chromosome 1p). This double elimination would allow a better prognosis and may be associated with increased response to alkylating agents in anaplastic astrocytoma and anaplastic oligodendroglioma. This component is present in about 10% of GB (apparently within the proneural and classical subtypes), however, its prognostic value in GB has not been demonstrated.

Thus, we conclude that there is still a need for useful biomarkers to determine, among other things, the response to temozolomide, radiotherapy, or the combination of both, in patients with GB, as well as potential new drugs for the treatment of these tumor types, such as immunotherapy.

### Brief description of the figures

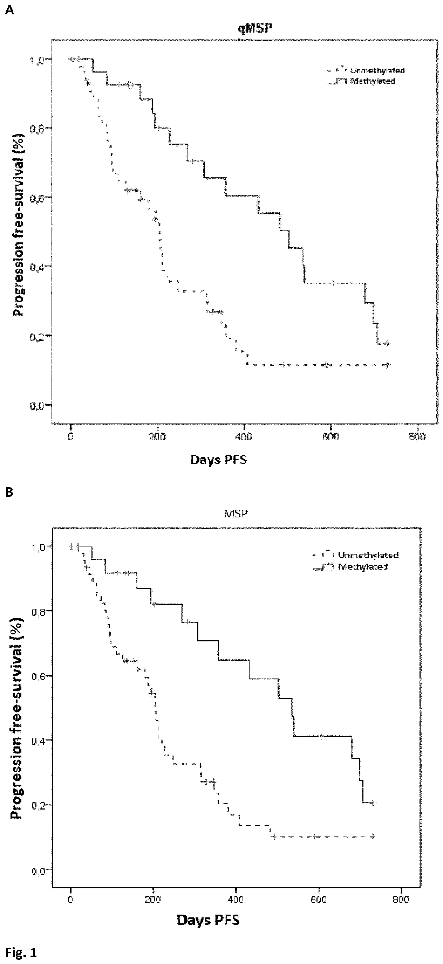

Figure 1. A: Kaplan-Meier curve to predict progression-free survival (PFS) in patients with grade IV gliomas treated with Temodal, using the standard MSP technique (PCR of specific methylation (N = 75, U (not methylated) = 48, M (methylated) = 27). B: Kaplan-Meier curve to predict progression-free survival (PFS) in patients with grade IV gliomas treated with Temodal, using the quantitative technique qMSP (quantitative Methylation-Specific PCR) (N = 75, U (unmethylated or non-methylated) = 45, M (methylated)= 30).

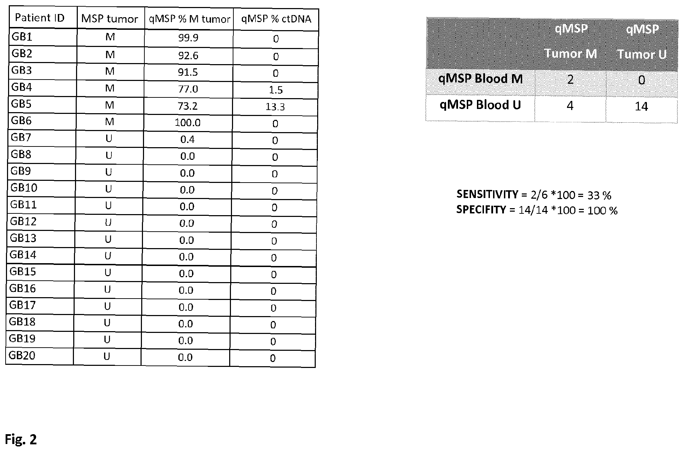

Figure 2. A: Percentage of methylation observed by qMSP for paired samples (tumour and free circulating DNA in plasma). B: Sensitivity (S) and specificity (E) obtained for the qMSP technique in blood. M (Methylated), U (unmethylated or non-methylated).

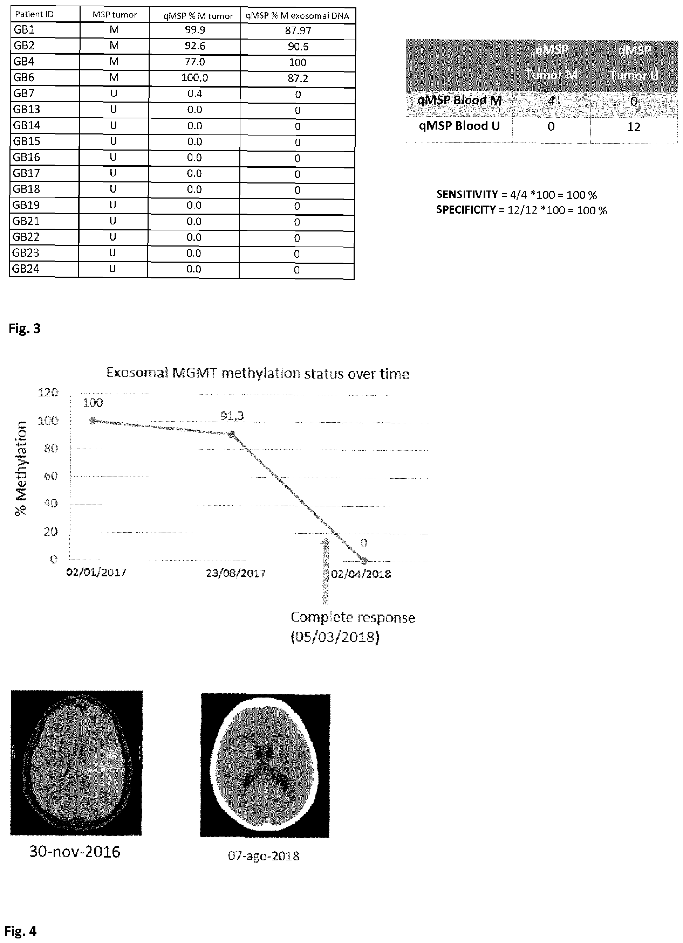

Figure 3: A: Percentage of methylation observed by qMSP for paired samples (tumour and DNA from plasma circulating exosomes). B: Sensitivity (S) and specificity (E) obtained for the qMSP technique in blood. M (Methylated), U (unmethylated or non-methylated).
Figure 4. Liquid biopsy follow-up of MGMT methylation in circulating exosomes of a patient. The presence of methylation in blood decreases as the patient responds to treatment. The magnetic resonance imaging (MRI) performed on 05/03/2018 (March 5^{th}, 2018) showed a complete radiological response of the tumour. One month later, methylation in blood is not detected.

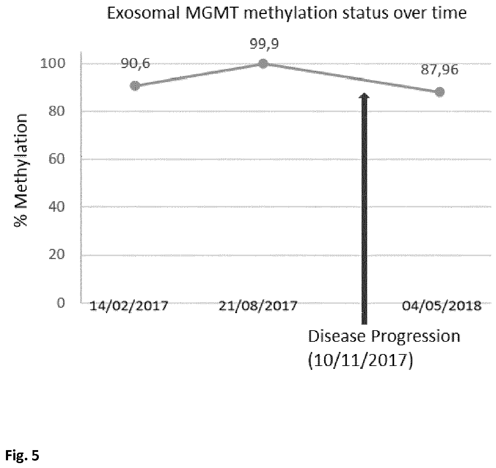

Figure 5. Liquid biopsy follow-up of MGMT gene methylation levels in DNA obtained from the plasma exosomal content of the same patient before the onset of treatment and two subsequent follow-up samples of the same patient. In this patient there was no response to temodal treatment, and tumor progression was observed on November 10, 2017. Methylation levels in DNA of exosomal origin assessed by qMSP in the initial and follow-up samples in this patient were always above 87%, even 5 months after observation of progression, indicating in this case that the continued detection of MGMT methylation presence in the exosomal content is related to the maintained presence of the disease.

### Detailed description of the invention

In the present invention a methodology has been identified for the detection of MGMT methylation in blood, serum or plasma with high sensitivity and specificity, surpassing the techniques described so far. This methodology will allow the diagnosis and monitoring of those patients diagnosed with glioblastomas using a non-invasive method. This invention could be extended to other tumor types in which methylation of the MGMT gene promoter was also present as a tumor marker.

To illustrate and demonstrate what was stated in the previous paragraph, we refer to examples 2 to 5 of this specification. Example 2 shows the values of the analysis of the presence of methylation valued by MSP and qMSP in a sample of tumour tissue and in a sample of non-exosomal circulating tumour DNA (tumour and circulating free DNA in plasma), where both samples are obtained from each patient, in a total of 20 patients. When qMSP amplification is performed in the plasma circulating free DNA not contained in the exosomal compartment, the sensitivity for detecting the presence of methylation in blood in those patients in whom the MGMT gene promoter in the tumor DNA is known to be methylated is only 25%, although specificity is maintained at 100% (see Figure 2). On the other hand, as illustrated in example 3, if the presence of methylation is determined in a tumour sample and in a sample of circulating tumour DNA from the exosomal content obtained from the same patient, by MSP and qMSP, in a total of 16 patients, when amplification by qMSP is carried out in the exosomal DNA, the sensitivity in its detection increases by 100%. That is, all patients with MGMT methylation in the tumor piece presented gene methylation in the blood exosomal content, maintaining specificity at 100% (see figure 3). In other words, the detection of MGMT methylation in blood can be performed with a high degree of sensitivity and specificity if it is performed from DNA molecules isolated or present inside the circulating exosomes obtained from this blood sample.

On the other hand, as illustrated in example 4, from the methylation levels of the MGMT gene in the DNA obtained from the plasma exosomal content of the same patient before the onset of treatment and two subsequent samples during the follow-up of the same patient, it has been determined that there is a clear concordance between the levels of methylated DNA for this gene at the time of diagnosis (100%), and the NMR image with the presence of the tumor. In liquid biopsy follow-up samples, a decrease in the amount of methylated DNA in the MGMT gene promoter region within the exosomal content is observed as the patient responds to the treatment, reaching values of 0%, which corresponds to an optimal response of the patient to treatment with Temodal. We can say, therefore, that the decrease of methylated MGMT levels in the exosomal content is related to a better response of the patient to the treatment, preferably to treatment with temozolomide, nitrosureas, procarbazine. On the other hand, as illustrated in example 5, the continuous detection of MGMT methylation presence in the exosomal content is related to the maintained presence of the disease and allows predicting its evolution.

Therefore, a first aspect of the invention refers to the *in vitro* use of DNA molecules isolated or present inside circulating exosomes of a sample isolated from serum, blood or plasma of a human subject to determine the percentage of methylation in that DNA of the O6-methylguanine-DNA methyltransferase (MGMT) gene promoter.

It is noted that the MGMT gene identified in the first aspect of this invention is located on chromosome band 10q26 and expresses a DNA reparative enzyme that eliminates alkyl groups from the O6 position of guanine, thus preventing base mismatching and cell death. In this way, MGMT protects the normal cell from carcinogenesis and also from the lethal effect of chemotherapy with alkylating agents. MGMT expression depends on the methylation status of its promoter, which lacks constituent regulatory elements and contains a CpG island including 97 CpGs which is known in the state of the art. Aberrant methylation of the cytokines of these dinucleotides leads to partial or total silencing of the gene, expressed in the decrease or absence of the MGMT protein, which would explain the increased susceptibility to chemotherapy.

It should be noted that the various methods for calculating the percentage of methylation in the DNA of the O6-methylguanine-DNA methyltransferase (MGMT) gene promoter from isolated DNA molecules are well known. Some of these methods are collected in the book Epigenetics Protocols by Trygve O. Tollefsbol Springer Science & Business Media, 2004 - 302 Seiten. In any case, a non-limiting way of carrying out this calculation or determination is by means of the formula: =100/(1+(2^(Ct_methylated-Ct_unmethylated))).

It should be noted that this invention allows the determination of the percentage of methylation in the DNA of the O6-methylguanine-DNA methyltransferase (MGMT) gene promoter from DNA molecules isolated or present in the inside of circulating exosomes of a sample isolated from serum, blood or plasma of a human subject, wherein such subject can be a healthy subject or a subject diagnosed with any type of glioblastoma, anaplastic astrocytoma or childhood brain tumour. Preferably, the subject is diagnosed with a glioblastoma, which can be a primary glioblastoma (GB1) or a secondary glioblastoma (GB2).

Preferably, the use of the first aspect of the invention is done for the purpose of diagnosing the presence or absence of a tumor in that subject. This is possible since it is known that the presence of hypermethylation in the DNA of the promoter of the O6-methylguanine-DNA methyl-transferase (MGMT) gene is frequently associated with neoplasia in human subjects, specifically colon cancer, lung cancer, head and neck cancer, ovary cancer, endometrium cancer, pancreas cancer, kidney cancer and spleen cancer as well as brain tumors such as glioblastoma.

More preferably, the use of the first aspect of the invention is done for the purpose of predicting progression-free survival in that subject or to predict the overall survival of the subject. More preferably, the subject suffers from glioblastoma, anaplastic astrocytoma or childhood brain tumour and has not been treated yet or is preferably being treated with a drug selected from the list consisting of: radiotherapy, immunotherapy, chemotherapy, a DNA alkylating agent, or any combination thereof.

More preferably, the use of the first aspect of the invention is done for the purpose of monitoring the disease of a subject who has glioblastoma, anaplastic astrocytoma or childhood brain tumour and who is preferably being treated with a drug selected from the list consisting of: radiotherapy, immunotherapy, chemotherapy, a DNA alkylating agent, or any combination thereof.

More preferably, the use of the first aspect of the invention is done in order to determine the response to treatment of a subject suffering from glioblastoma, anaplastic astrocytoma or childhood brain tumour and who is preferably being treated with a drug selected from the list consisting of: radiotherapy, immunotherapy, chemotherapy, a DNA alkylating agent, or any combination thereof.

Even more preferably, this alkylating agent, according to any of the above preferred embodiments, is selected from the list consisting of nitrogen mustards such as mechlorethamine, cyclophosphamide, ifosfamide, melphalan, or chlorambucil; ethyleneimines and methylmelamines such as altretamine; methylhydrazine derivatives such as procarbazine; alkyl sulfonates such as busulfan; nitrosoureas such as carmustine; triazenes such as dacarbazine or temozolomide; or platinum coordination complexes such as cisplatin, carboplatin, or oxaliplatin. More preferably said alkylating agent is temozolomide.

In a particular embodiment of the first aspect of the invention or any of its preferred embodiments, such circulating exosomes from a sample isolated from serum, blood, or plasma are of tumor origin. In this sense, it should be noted that there are specific kits that allow DNA from circulating exosomes to be obtained from a sample isolated from serum, blood or plasma of tumour origin (Exosomics).

A second aspect of the invention refers to an *in vitro* method to determine the methylation percentage of the O6-methylguanine-DNA methyltransferase (MGMT) gene promoter which comprises determining, from circulating exosomes of a serum, blood or plasma sample isolated from a human subject, the methylation percentage of the O6-methylguanine-DNA methyltransferase (MGMT) gene promoter of the DNA molecules isolated or present within these circulating exosomes.

In a particular embodiment of the second aspect of the invention, these circulating exosomes are tumour circulating exosomes.

In another particular embodiment of the second aspect of the invention, such determination is performed by Methylation-Specific PCR (MSP), quantitative Methylation-Specific PCR (qMSP), or by pyrosequencing techniques. It should be noted that in order to determine the percentage of methylation in the DNA of the O6-methylguanine-DNA methyltransferase (MGMT) gene promoter from DNA molecules isolated or present inside circulating exosomes, the quantitative Methylation-Specific PCR (qMSP) technique is used in a particularly preferred manner. Both the Methylation-Specific PCR (MSP) and the quantitative Methylation-Specific PCR (qMSP) techniques are explained in the examples included in this patent specification.

A third aspect of the invention refers to a method to monitor the oncological disease of a subject diagnosed with a glioblastoma, anaplastic astrocytoma or childhood brain tumour, preferably to monitor the disease of a subject diagnosed with a glioblastoma which in turn can be a primary glioblastoma (GB1) or a secondary glioblastoma (GB2), wherein such a method comprises the following steps:
a. Performing the methodology to determine the percentage of methylation of the promoter of the O6-methylguanine-DNA methyltransferase (MGMT) gene according to the second aspect of the invention; and
b. Determining the subject's disease monitoring or progression based on the percentage of methylation determined in step a).

It is noted that with respect to the third aspect of the invention, the disease of the subject is monitored based on the percentage of methylation determined in step a), wherein a lower percentage, a significant reduction of DNA methylation with respect to the percentage of methylation of the promoter of a sample obtained from the same subject previously or with respect to a reference value, is indicative of favorable response to treatment. On the other hand, a higher percentage of methylation, a significant increase of DNA methylation with respect to the percentage of methylation of the promoter in a sample obtained from the same subject previously or with respect to a reference value, is indicative of an unfavourable response to treatment.

A fourth aspect of the invention refers to a method for predicting the response to treatment of a subject treated with a drug selected from the list consisting of: radiotherapy, immunotherapy, chemotherapy, DNA alkylating agent, or any combination thereof, wherein such subject is diagnosed with a glioblastoma, anaplastic astrocytoma or childhood brain tumour, wherein the subject is preferably diagnosed with a glioblastoma which in turn can be a primary glioblastoma (GB1) or a secondary glioblastoma (GB2), and wherein such method comprises the following steps:
a. Performing the methodology to determine the percentage of methylation of the promoter of the O6-methylguanine-DNA methyltransferase (MGMT) gene according to the second aspect of the invention; and
b. Determining the response to treatment of the subject's disease based on the percentage of methylation determined in step a).

It is noted that with respect to the fourth aspect of the invention, if the intent is to determine patient's response to treatment before the onset of treatment, a higher percentage of DNA methylation with respect to a healthy control subject or with respect to a reference value is indicative of a potential favorable disease response to treatment with an alkylating agent, radiation therapy, immunotherapy, chemotherapy, or any combination thereof; and wherein once treatment has commenced the gradual decrease in methylation is indicative of a favorable tumor response to treatment.

A fifth aspect of the invention refers to a method to diagnose the presence or absence of a tumor, in particular this diagnostic method allows to identify the presence of methylation in the DNA of the O6-methylguanine-DNA methyltransferase (MGMT) gene promoter and therefore the potential presence of a neoplasm frequently associated with the hypermethylation of this promoter as colon cancer, lung cancer, head and neck cancer, ovary cancer, endometrium cancer, pancreas cancer, kidney cancer and spleen cancer as well as brain tumors such as glioblastoma; wherein this method comprises the following steps:
a. Performing the methodology to determine the percentage of methylation of the promoter of the O6-methylguanine-DNA methyltransferase (MGMT) gene according to the second aspect of the invention; and
b. Determining the potential presence of a neoplasm frequently associated with this type of hypermetilation in the subject based on the percentage of methylation determined in step a).

Preferably, in the method according to any of the second to fourth aspects, this alkylating agent is selected from the list consisting of nitrogen mustards such as mechlorethamine, cyclophosphamide, ifosfamide, melphalan, or chlorambucil; ethyleneimines and methylmelamines such as altretamine; methylhydrazine derivatives such as procarbazine; alkyl sulfonates such as busulfan; nitrosoureas such as carmustine; triazenes such as dacarbazine or temozolomide; or platinum coordination complexes such as cisplatin, carboplatin, or oxaliplatin. More preferably said alkylating agent is temozolomide.

A sixth aspect of the invention refers to a kit comprising the reagents necessary to perform the Methylation-Specific PCR (MSP) technique, preferably quantitative Methylation-Specific PCR (qMSP), to implement the method of any of the second to fifth aspects of the invention. Preferably, such reagents comprise specific primers for the non-methylated MGMT promoter and the methylated MGMT promoter. More preferably, the specific primers for the non-methylated (or unmethylated) MGMT promoter are: um_MGMT: F: TTTGTGTTTTGATGTTTGTAGGTTTTTGT; and R:AACTCCACACTCTTCCAAAAACAAAACA; and for the methylated MGMT promoter are: m_MGMT: F: TTTCGACGTTCTAGGTTTTCGC; and R: GCACTCTTCCGAAAACGAAACG. Likewise, and preferably, in order to be able to implement the quantitative Methylation-Specific PCR (qMSP), this method also comprises two labeled probes, for example with fluorochromes, to specifically identify modified and methylated DNA such as Probe M:6FAM-CAAATCGCAAACGATA-MGB-NFQ and modified and unmethylated DNA such as Probe U:VIC5-CAAATCACAAACAATA-MGB-NFQ.

A seventh aspect of the invention refers to the use of the kit of the sixth aspect of the invention to implement the methodology according to any of the second to fifth aspects.

The following examples are merely illustrative of the present invention.

### Examples

### Materials and methods

In the evaluation of MGMT promoter methylation in the following examples, the DNA of glioblastoma tumors is subjected to bisulphite reaction, whereby non-methylated cytosines residues are converted to uracils after treatment. Peripheral blood healthy lymphocytes methylated *in vitro* with the enzyme CpG-Methyltransferase (M.Sssl) are used as positive control of methylation. The modified DNA is pre-amplified (bisulphite amplification, BS) with a pair of primers that does not discriminate between methylated and non-methylated DNA and amplifies a size of 274 base pairs (MGMT_BS:F GGATATGTTGGGATAGTT, MGMT_BS:R: CCGAAAAAAAACTAAACAACACCT) illustrated below, at 56ºC and 36 cycles.
Genomic starting position: Chr10:131265476
Genomic end position: Chr10:131265750
Amplicon 274 base pairs 
‘5tgcccCTCGGCCCCGCCCCCGCGCCCC(g.131,265,476)GGATATGCTGGGACAGCCCG
CGCCCCTAGAACGCTTTGCGTCCCGACGCCCGCAGGTCCTCGCGGTGCGCACCGTTTG
 
CGACTTGgtgagtgtctgggtcgcctcgctcccggaagagtgcggagctctccctcgg
gacggtggcagcctcgagtggtcctgcaggcgccctcacttcgccgtcgggtgtgggg
ccgccctgacccccacccatcccgggcgagctccaggtgcgccccaagtgcctcccag
gtgttgcccagcctttccccgg(g.131,265,750)gcctggggttc-3'.
 Bold: part of the UTR region
Underlined: exon and intron primers in Ensembl according to GRCh37 - 131,265,448-131,265,560
Black: First exon
Grey: part of the first intron.
Probes: Underlined and italic region, containing 2 CG.

In the case of DNA from circulating exosomes, a different reverse primer is used to amplify a shorter fragment of 166 base pairs (MGMT_BS:R2 CCTACAAAACCACTCRAAACT) at 56ºC and 36 cycles. For the analysis of MGMT methylation in tissue, MSP and qMSP are performed, while for plasma amplifications, only qMSP is used. For MSP reactions, PCRs are performed independently for methylated and non-methylated alleles with specific primers for the unmethylated MGMT promoter (um_MGMT: F: TTTGTGTTTTGATGTTTGTAGGTTTTTGT; R.: AACTCCACACTCTTCCAAAAACAAAACA) and the methylated MGMT promoter (m_MGMT: F: TTTCGACGTTCTAGGTTTTCGC; R: GCACTCTTCCGAAAACGAAACG) and under variable conditions for ringing temperatures (55-66 ºC, 35 and 37 cycles respectively). The reaction products are analyzed by electrophoresis in agarose or acrylamide gels. For qMSP reactions the same 4 previous primers are maintained and two fluorochrome-labeled probes are introduced to quantify the % of methylated molecules in the sample and to increase the sensitivity of the technology. ProbeM: 6FAM-CAAATCGCAAACGATA-MGB-NFQ and modified and non-methylated DNA ProbeU: VIC5-CAAATCACAAACAATA-MGB-NFQ.

### 1. Kaplan-Meier curve to predict progression-free survival (PFS) in patients with grade IV gliomas treated with Temodal.

Paraffin tissue samples from patients with tumor cellularity selection above 50% were cut at 10 microns, these cuts were dewaxed with Xylol, digested with K proteinase and SDS and the DNA was subsequently extracted by the phenol-chloroform method, in which two centrifugations with phenol:Chloroform:lsoamyl alcohol were performed for 5 minutes at 14,000 rpm, followed by a centrifugation with chloroform. The DNA was then precipitated with cold alcohol and glycogen as the vehicle. It is left at -20ºC overnight, centrifuged for 45 minutes at 140000C at 4ºC and re-suspended in TE.

Subsequently, approximately 1 ug of the extracted DNA was modified by bisulphite and hydroquinone to maintain methylation signals in cytosines. It was then purified using the Wizard DNA Clean-Up (Promega) system. The modification was completed by incubation with soda (NaOH) and precipitation with ammonium acetate, glycogen and ethanol. It should be noted that this modification by bisulphite could have been carried out by any kit available on the market for this purpose, such as the various kits from Zymo (EZ DNA methylation, Methylation-Gold, Methylation-lightening, methylation-Direct, etc.). For the present example it has been carried out by manual protocol since it is considered more efficient when modifying DNA.

Subsequently the samples were pre-amplified. The modified and pre-amplified DNA was used to assess the methylation presence of the MGMT gene promoter with two different techniques (MSP and qMSP) as illustrated in the *Materials and Methods* section. Briefly, each Master mix included primers, modified DNA, DNTPs, polymerase enzyme buffer, and polymerase enzyme. Two PCR reactions were then performed for each patient sample and these samples were run in a 6% acrylamide gel. The presence or absence of the amplified fragment was determined in the reaction performed with the primers to identify the methylated DNA. If the amplification was positive this meant the presence of methylation in the analyzed sample. On the other hand, the presence of the band or fragment amplified with the primers that identify the non-methylated DNA, was significant of correct DNA modification and development of the technique, since all tumors contain non-methylated DNA and this amplification must always take place for any sample.

From the results obtained and as illustrated in Figure 1, a Kaplan-Meier Curve was performed to predict progression-free survival (PFS) in 75 patients with grade IV gliomas treated with Temodal, using the standard MSP technique (Methylation-Specific PCR) (N = 75, U = 48, M = 27) as illustrated in the previous paragraph. 27 patients presented methylation and 48 resulted in a non-methylated sample. The graph on the right of figure 1 represents the survival curves in the same cohort of patients with the quantitative technique qMSP (quantitative Methylation-Specific PCR, with four primers and two probes) (N = 75, U (not methylated) = 45, M (methylated) = 30). In both cases we observed that patients where MGMT promoter was methylated have a longer disease-free survival than patients where MGMT promoter was unmethylated. In addition, the quantitative technique reproduces the standard technique, and even rescues 3 patients who were considered non-methylated by MSP. These results allow us to apply this technique, of greater sensitivity when the DNA is compromised in quantity, as is the case of its blood levels.

### 2. Percentage of methylation observed by qMSP for paired samples (tumour and free circulating DNA in plasma).

This example, the results of which are illustrated in Figure 2, shows the values of the analysis of the presence of methylation assessed by MSP and qMSP in a sample of tumour tissue and in the circulating non-exosomal tumour DNA (tumour and circulating free DNA in plasma), where both samples are obtained from each patient, in a total of 20 patients.

When qMSP amplification is made in the plasma circulating free DNA not contained in the exosomal compartment, the sensitivity for detecting the presence of methylation in blood in patients in whom the MGMT promoter of the tumor sample is known to be methylated is only 25%, although specificity is maintained at 100%.

To conduct this experiment, blood samples from patients were processed within the first hour after collection. They were centrifuged at 5130 rpm for 10 minutes at 4ºC, plasmas were aliquoted and then centrifuged for 20 minutes at 14000 rpm at 4ºC and aliquoted for subsequent storage at -80ºC. The circulating free DNA was extracted, modified and pre-amplified as explained in the previous example, using the primer reverse2 which allows amplification of a shorter fragment. Subsequently, the methylation levels of the MGMT gene were assessed by qMSP with four primers and two probes in the same reaction.

According to the data obtained, we observe that only 2 of the 8 patients with methylated tumor for the MGMT gene are identified in circulating plasma tumor DNA.

### 3. Percentage of methylation observed by qMSP for paired samples (tumour and DNA from circulating exosomes in plasma).

This example shows the values of the analysis of the presence of methylation in a tumor sample and in a sample of circulating tumor DNA of the exosomal content obtained from the same patient, by MSP and qMSP in a total of 16 patients. When qMSP amplification is performed in the exosomal DNA, the sensitivity in its detection increases by 100%. All patients with MGMT methylation in the tumor piece presented gene methylation in the blood exosomal content. The specificity was maintained at 100%.

All blood samples from patients were processed within the first hour after collection. They were centrifuged at 5130 rpm for 10 minutes at 4ºC, plasmas were aliquoted and then centrifuged for 20 minutes at 14C at 4ºC and aliquoted for storage at -80ºC. Circulating exosomes were extracted from the plasma, and then the DNA was extracted from that exosomal content. The DNA was modified and pre-amplified using the primer reverse2 which allows amplification of a shorter fragment. The methylation levels of the MGMT gene were then assessed by qMSP with four primers and two probes in the same reaction. Figure 3 illustrates how 4 out of 4 patients with methylated tumor for the MGMT gene are identified using tumor DNA of exosomal origin in plasma. Sensitivity (S) and specificity (E) obtained for the qMSP technique in blood. M (Methylated), U (unmethylated).

### 4. Liquid biopsy follow-up of MGMT methylation in circulating exosomes of a patient.

This example shows the methylation levels of the MGMT gene in DNA obtained from the plasma exosomal content of the same patient prior to the onset of treatment and two subsequent follow-up samples of the same patient. As illustrated in Figure 4, there is a clear concordance between the levels of methylated DNA for this gene at the time of diagnosis (100%), and the NMR image with the presence of the tumor. In liquid biopsy follow-up samples, a decrease in the amount of methylated DNA in the MGMT gene promoter region within the exosomal content is observed as the patient responds to treatment, reaching 0% values, which corresponds to an optimal response of the patient to treatment with Temodal. The magnetic resonance imaging (MRI) performed on 05/03/2018 (March 5^{th}, 2018) showed a complete radiological response of the tumour. One month later, blood methylation was not detected. We can therefore say that the decrease in levels of methylated MGMT in the exosomal content is related to a better patient response.

### 5. Liquid biopsy follow-up of MGMT methylation in circulating exosomes of a patient.

This example shows the methylation levels of the MGMT gene in the DNA obtained from the plasma exosomal content of one patient before the onset of treatment and two subsequent follow-up samples of the same patient (see figure 5). In this patient there was no response to temodal treatment, and in fact tumor progression was observed on November 10, 2017. Methylation levels in DNA of exosomal origin assessed by qMSP in the initial and follow-up samples in this patient were always above 87%, even 5 months after observation of progression, indicating in this case that the continued detection of the presence of MGMT methylation in the exosomal content is related with the continued presence of the disease and the lack of therapeutic response.

## Claims

1. *In vitro* use of DNA molecules isolated or present within circulating exosomes from a sample isolated from serum, blood or plasma of a human subject to determine the percentage of methylation in that DNA of the promoter of the O6-methylguanine-DNA methyltransferase (MGMT) gene.

2. *In vitro* use according to claim 1, wherein such subject is diagnosed with glioblastoma.

3. Use according to claim 2, wherein such subject is diagnosed with a primary glioblastoma (GB1) or with a secondary glioblastoma (GB2).

4. *In vitro* use according to any of claims 2 to 3, wherein such determination is performed to monitor the progression of glioblastoma in that subject, wherein that subject is being treated with a treatment selected from the list consisting of: radiation therapy, immunotherapy, chemotherapy, DNA alkylating agent, or any combination thereof.

5. *In vitro* use according to claim 4, wherein such determination is performed to predict the progression-free survival in that subject or to predict the overall survival of the subject.

6. *In vitro* use according to any of claims 2 to 3, wherein such determination is performed to predict the response to treatment in that subject, wherein that subject is being treated with a treatment selected from the list consisting of: radiotherapy, immunotherapy, chemotherapy, DNA alkylating agent, or any combination thereof.

7. *In vitro* use according to any of claims 2 to 3, wherein such determination is performed to identify the presence or absence of a tumor whose presence is associated with hypermethylation in the DNA of the promoter of the O6-methylguanine-DNA methyltransferase (MGMT) gene.

8. *In vitro* use according to any of claims 4 to 6, wherein such subject is being treated with an alkylating agent that is selected from the list consisting of: nitrogen mustards such as mechlorethamine, cyclophosphamide, ifosfamide, melphalan, or chlorambucil; ethylenimines and methylmelamines such as altretamine; methylhydrazine derivatives such as procarbazine; alkyl sulfonates such as busulfan; nitrosoureas such as carmustine; triazenes such as dacarbazine or temozolomide; or platinum coordination complexes such as cisplatin, carboplatin, or oxaliplatin. More preferably said alkylating agent is temozolomide.

9. Use according to claim 8, wherein such alkylating agent is temozolomide.

10. Use according to any of claims 1 to 8, wherein such exosomes are of tumor origin.

11. *In vitro* method to determine the methylation percentage of the O6-methylguanine-DNA methyltransferase (MGMT) gene promoter which comprises determining, from circulating exosomes of a serum, blood or plasma sample isolated from a human subject, the methylation percentage of the O6-methylguanine-DNA methyltransferase (MGMT) gene promoter of the DNA molecules isolated or present within these circulating exosomes.

12. The method according to claim 11, wherein these circulating exosomes are tumour circulating exosomes.

13. The method according to any of claims 11 or 12, wherein such determination is performed by Methylation-Specific PCR (MSP) techniques, preferably qMSP, or by pyrosequencing.

14. The method according to claim 13, wherein the technique is qMSP, and PCRs are performed independently for methylated and non-methylated alleles with specific primers for the non-methylated MGMT promoter and the methylated MGMT promoter, and wherein the reaction products are preferably analyzed by electrophoresis in agarose or acrylamide gels; and wherein the method also comprises two labeled probes to specifically identify modified and methylated DNA and modified and non-methylated DNA.

15. Method to monitor the oncological disease of a subject diagnosed with a glioblastoma, wherein this method comprises the following steps:
a. Performing the methodology to determine the methylation percentage of the O6-methylguanine-DNA methyltransferase (MGMT) gene promoter according to any of claims 11 to 14; and
b. Determining the subject's disease monitoring or progression based on the percentage of methylation determined in step a);
c. Wherein a significant reduction in DNA methylation with respect to the promoter methylation percentage of a sample previously obtained from the same subject or with respect to a reference value is indicative of favorable subject progression.

16. A method for predicting the response to treatment of a subject treated with a drug selected from the list consisting of: radiation therapy, immunotherapy, chemotherapy, DNA alkylating agent, or any combination thereof, wherein the subject is diagnosed with a glioblastoma, and wherein such method comprises the following steps:
a. Performing the methodology to determine the methylation percentage of the O6-methylguanine-DNA methyltransferase (MGMT) gene promoter according to any of claims 11 to 14; and
b. Determining the response to treatment of the subject's disease based on the percentage of methylation determined in step a);
c. Where if the intent is to determine patient's response to treatment prior to the onset of treatment, a higher percentage of DNA methylation with respect to a healthy control subject or with respect to a reference value is indicative of a potential favorable disease response to treatment with an alkylating agent, radiation therapy, immunotherapy, chemotherapy, or any combination thereof; and wherein once treatment is initiated the gradual decrease in methylation is indicative of a favorable tumor response to treatment.

17. Method to diagnose the presence of a neoplasm associated with the hypermethylation of the O6-methylguanine-DNA methyltransferase (MGMT) gene promoter in a subject, wherein this method comprises the following steps:
a. Performing the methodology to determine the methylation percentage of the O6-methylguanine-DNA methyltransferase (MGMT) gene promoter according to any of claims 11 to 14; and
b. Determining the potential presence of a neoplasm frequently associated with this type of hypermethylation of the MGMT gene promoter in the subject based on the percentage of methylation determined in step a).

18. Kit comprising specific primers for the non-methylated MGMT promoter and the methylated MGMT promoter, selected from the list consisting of specific primers for the unmethylated MGMT promoter um_MGMT: F: TTTGTGTTTTGATGTTTGTAGGTTTTTGT; and R:AACTCCACACTCTTCCAAAAACAAAACA and for the methylated MGMT promoter: m_MGMT: F: TTTCGACGTTCTAGGTTTTCGC; and R: GCACTCTTCCGAAAACGAAACG.

19. The kit according to claim 18, wherein said kit also comprises fluorochrome-labeled probes suitable for specifically identifying modified and methylated DNA such as ProbeM:6FAM-CAAATCGCAAACGATA-MGB-NFQ and modified and non-methylated DNA such as ProbeU:VIC5-CAAATCACAAACAATA-MGB-NFQ.

20. Use of the kit according to any of claims 18 or 19, to implement the methodology according to any of claims 11 to 17.

21. *In vitro* use of a kit comprising the reagents necessary to perform the Methylation-Specific PCR (MSP) technique, preferably qMSP, to implement the method according to any of Claims 11 to 17.
